(19) Europäisches Patentamt
European Patent Office
Office européen des brevets

(11) Veröffentlichungsnummer: **0 217 197 B1**

(12) **EUROPÄISCHE PATENTSCHRIFT**

(45) Veröffentlichungstag der Patentschrift: **16.10.91**

(51) Int. Cl.$^5$: **G01N 33/72**

(21) Anmeldenummer: **86112620.9**

(22) Anmeldetag: **12.09.86**

# (54) Bestimmung von Bilirubin und entsprechende Reagentien.

(30) Priorität: **26.09.85 CH 4172/85**

(43) Veröffentlichungstag der Anmeldung:
**08.04.87 Patentblatt 87/15**

(45) Bekanntmachung des Hinweises auf die Patenterteilung:
**16.10.91 Patentblatt 91/42**

(84) Benannte Vertragsstaaten:
**AT BE CH DE FR GB IT LI NL SE**

(56) Entgegenhaltungen:
**EP-A- 0 087 150**
**WO-A-83/03254**
**US-A- 4 078 892**
**US-A- 4 119 401**
**US-A- 4 246 133**

(73) Patentinhaber: **F. HOFFMANN-LA ROCHE AG**
**Postfach 3255**
**CH-4002 Basel(CH)**

(72) Erfinder: **Le Dain, Michel, Dr.**
**10 rue Jean Schoerlin Neuwiller**
**F-68220 Hegenheim(FR)**

(74) Vertreter: **Kloter, Rudolf et al**
**Postfach 3255 Grenzacherstrasse 124**
**CH-4002 Basel(CH)**

EP 0 217 197 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Bestimmung von Gesamt-Bilirubin in einer biologischen Probe nach der Diazomethode sowie hierfür geeignete Reagenzien.

Die Bestimmung von Gesamt-Bilirubin nach der Diazomethode ist eine dem Fachmann bestens bekannte Technik. Allerdings weisen diese Methode sowie die hierfür verwendeten Reagenzien einige Nachteile auf, insbesondere eine tiefe Linearität sowie eine komplizierte Reagenzienzubereitung.

Die Methode gemäss vorliegender Erfindung bzw. die hierfür vorgesehenen Reagenzien ergeben einen Test, der sich durch die nachfolgenden überraschenden Vorteile auszeichnet:

- höhere Linearität
- einfachere Reagenzienzubereitung
- lange Gebrauchslösungsstabilität und
- gute Präzision und gute Reproduzierbarkeit.

Zudem erlaubt die günstige Kinetik der Reaktion die Anwendung des Autoblanking-Verfahrens.

Bekanntlich besteht ja die Diazomethode zur Bestimmung von Bilirubin darin, dass das in der biologischen Probe enthaltene Bilirubin mit einer DiazoniumVerbindung zu einem Farbstoff reagiert, dessen Intensität der Menge an Bilirubin in der Probe entspricht.

In der EP-A-0 087 150 wird vorgeschlagen, die Bestimmung von Gesamt-Bilirubin nach der Diazomethode in Gegenwart des Akzelerators Tetramethylensulfon durchzuführen. Die Gegenwart von Tetramethylensulfon erlaubt zudem die Verwendung von nicht-ionischen oberflächenaktiven Mitteln. Die beschriebene Methode ergibt lineare Resultate bis zu einem Gehalt von 20 mg/dl Bilirubin.

Demgegenüber wurde im Rahmen der vorliegenden Erfindung nun gefunden, dass die eingangs erwähnten Vorteile dadurch erzielt werden können, wenn man bei der BilirubinBestimmung nach der Diazomethode ein oder mehrere Detergenzien der allgemeinen Formel

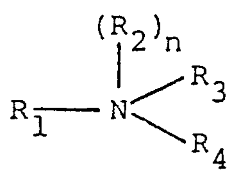

I

worin $R_1$ gerades oder verzweigtes Alkyl mit 10-16 C-Atomen bedeutet, $R_2$ Sauerstoff oder gerades oder verzweigtes Alkyl mit 1-4 C-Atomen bedeutet; $R_3$, sofern $n = 0$ ist, Wasserstoff oder gerades oder verzweigtes Alkyl mit 1-4 C-Atomen oder $-(CH_2-CH_2-O)_xH$ bedeutet, wobei x eine ganze Zahl von 1-5 ist, $R_3$, sofern $n = 1$ und $R_2$ Sauerstoff bedeutet, gerades oder verzweigtes Alkyl mit 1-4 C-Atomen oder $-(CH_2-CH_2-O)_xH$ bedeutet, wobei x eine ganze Zahl von 1-5 ist, $R_3$, sofern $n = 1$ und $R_2$ gerades oder verzweigtes Alkyl mit 1-4 C-Atomen bedeutet, gerades oder verzweigtes Alkyl mit 1-4 C-Atomen bedeutet; $R_4$, sofern $n = 0$ ist, Wasserstoff oder gerades oder verzweigtes Alkyl mit 1-4 C-Atomen oder $-(CH_2-CH_2-O)_xH$ bedeutet, wobei x eine ganze Zahl von 1-5 ist, $R_4$, sofern $n = 1$ und $R_2$ Sauerstoff bedeutet, gerades oder verzweigtes Alkyl mit 1-4 C-Atomen, oder $-(CH_2-CH_2-O)_xH$ bedeutet, wobei x eine ganze Zahl von 1-5 ist, $R_4$, sofern $n = 1$ und $R_2$ gerades oder verzweigtes Alkyl mit 1-4 C-Atomen, gerades oder verzweigtes Alkyl mit 1-10 C-Atomen bedeutet; oder $R_2$, $R_3$ und $R_4$ zusammen mit dem Stickstoffatom eine Pyridiniumgruppe bilden, wobei in diesem Fall, sowie im Falle, dass $n = 1$ und $R_2$ gerades oder verzweigtes Alkyl mit 1-4 C-Atomen bedeutet, ein entsprechendes Anion vorhanden ist,
verwendet.

Bevorzugte Detergenzien (im nachfolgenden auch als Acceleratorreagenzien bezeichnet) der Formel I sind N,N-Dimethyl-decylamin, N,N-Dimethyl-dodecylamin, N,N-Dimethyl-tetradecylamin, N,N-Dimethyl-hexadecylamin, Didecyldimethylammoniumchlorid, Dodecyltrimethylammoniumchlorid, Dodecyltrimethylammoniumbromid, Dodecyläthyldimethylammoniumbromid, Dodecylpyridiniumchlorid, Tetradecyltrimethylammoniumbromid sowie N,N-Dimethyldodecylaminoxid. Ein ganz besonders bevorzugtes Detergenz der allgemeinen Formel I ist Dodecyläthyldimethylammoniumbromid.

Nach einem ersten Aspekt betrifft die vorliegende Erfindung ein wässriges Reagenz zur Bestimmung von Gesamt-Bilirubin in einer biologischen Probe enthaltend einerseits

a) ein oder mehrere Detergenzien der allgemeinen Formel

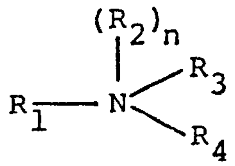

I

worin $R_1$ gerades oder verzweigtes Alkyl mit 10-16 C-Atomen bedeutet, $R_2$ Sauerstoff oder gerades oder verzweigtes Alkyl mit 1-4 C-Atomen bedeutet; $R_3$, sofern n =0 ist, Wasserstoff oder gerades oder verzweigtes Alkyl mit 1-4 C-Atomen oder $-(CH_2-CH_2-O)_xH$ bedeutet, wobei x eine ganze Zahl von 1-5 ist, $R_3$, sofern n = 1 und $R_2$ Sauerstoff bedeutet, gerades oder verzweigtes Alkyl mit 1-4 C-Atomen oder $-(CH_2-CH_2-O)_xH$ bedeutet, wobei x eine ganze Zahl von 1-5 ist, $R_3$, sofern n = 1 und $R_2$ gerades oder verzweigtes Alkyl mit 1-4 C-Atomen bedeutet, gerades oder verzweigtes Alkyl mit 1-4 C-Atomen bedeutet; $R_4$, sofern n = 0 ist, Wasserstoff oder gerades oder verzweigtes Alkyl mit 1-4 C-Atomen oder $-(CH_2-CH_2-O)_xH$bedeutet, wobei x eine ganze Zahl von 1-5 ist, $R_4$, sofern n = 1 und $R_2$ Sauerstoff bedeutet, gerades oder verzweigtes Alkyl mit 1-4 C-Atomen, oder $-(CH_2-CH_2-O)_xH$ bedeutet, wobei x eine ganze Zahl von 1-5 ist, $R_4$, sofern n = 1 und $R_2$ gerades oder verzweigtes Alkyl mit 1-4 C-Atomen, gerades oder verzweigtes Alkyl mit 1-10 C-Atomen bedeutet; oder $R_2$, $R_3$ und $R_4$ zusammen mit dem Stickstoffatom eine Pyridiniumgruppe bilden, wobei in diesem Fall, sowie im Falle, dass n = 1 und $R_2$ gerades oder verzweigtest Alkyl mit 1-4 C-Atomen bedeutet, ein entsprechendes Anion vorhanden ist,

b) eine aromatische Aminoverbindung, die sich mit Nitrit in eine bei der Bilirubinbestimmung übliche Diazoniumverbindung überführen lässt,

c) eine starke Säure, und andererseits

d) eine wässrige Nitritlösung.

Nach einem zweiten Aspekt betrifft die vorliegende Erfindung ein wässriges Reagenz zur Bestimmung von Gesamt-Bilirubin in einer biologischen Probe enthaltend

a) ein oder mehrere Detergenzien der allgemeinen Formel I,

b) eine bei der Bilirubinbestimmung übliche Diazoniumverbindung und

c) eine starke Säure.

In den vorerwähnten wässrigen Reagenzien ist das Detergenz der allgemeinen Formel I bevorzugt in einer Konzentration von 0,25-10%, insbesondere in einer Konzentration von 1-3% vorhanden.

Die aromatische Aminoverbindung ist bevorzugt in einer Konzentration von 0,5-40 mM, besonders bevorzugt in einer Konzentration von 1-30 mM vorhanden.

Als bevorzugte starke Säuren kommen Amidoschwefelsäure, Salzsäure sowie Schwefelsäure in Betracht.

Die Konzentration an Amidoschwefelsäure liegt vorzugsweise im Bereich von 0,025-1 Mol/l, insbesondere in einem Bereich von 0,10-0,25 Mol/l und der pH des Reagenz ist vorzugsweise zwischen 1 und 3.

Bei Verwendung von Salzsäure ist die Konzentration bevorzugt im Bereich von 0,01-0,5 Mol/l, insbesondere im Bereich von 0,05-0,2 Mol/l, und der pH des Reagenz ist vorzugsweise 1-3.

Bei Verwendung von Schwefelsäure liegt die Konzentration vorzugsweise im Bereich von 0,01-0,5 Mol/l, insbesondere im Bereich von 0,05-0,25 Mol/l und der pH des Reagenz ist vorzugsweise zwischen 1 und 3.

Die aromatische Aminoverbindung, die sich mit der Nitritlösung zur entsprechenden Diazoniumverbindung umsetzen lässt, ist vorzugsweise 4-Aminobenzolsulfonsäure, 2,4- oder 2,5-Dichloranilin, 4-Nitroanilin, 2-Methoxy-4-nitroanilin oder 2-Methoxy-5-nitroanilin.

Die vorerwähnte Nitritlösung ist vorzugsweise in einer Konzentration von 4-100 Mol/l vorhanden.

Sofern das Reagenz bereits die fertige Diazoniumverbindung enthält, handelt es sich hierbei vorzugsweise um diazotierte Aminobenzolsulfonsäure oder diazotiertes 2,4- oder 2,5-Dichloranilin oder diazotiertes 4-Nitroanilin oder 2-Methoxy-4-nitroanilin oder 2-Methoxy-5-nitroanilin.

Die Konzentration an Diazoniumverbindung in der wässrigen Lösung liegt vorzugsweise im Bereich von 0,10-5,0 g/l, insbesondere in einer Konzentration von 0,15-3,0 g/l.

Die bevorzugten Detergenzien der allgemeinen Formel I wurden bereits eingangs erwähnt, doch kann hierzu noch bemerkt werden, dass die Substituenten $R_3$ und $R_4$ in der allgemeinen Formel I bevorzugt die gleiche Bedeutung haben.

Nach einem dritten Aspekt betrifft die vorliegende Erfindung ein stabilisiertes und lyophilisiertes Reagenz zur Bestimmung von Gesamt-Bilirubin in einer biologischen Probe enthaltend diazotierte Aminobenzolsulfonsäure, diazotiertes 4-Nitroanilin, 2-Methoxy-4-nitroanilin, 2-Methoxy-5-nitroanilin, 2,4-Dichloranilin oder 2,5-Dichloranilin, sowie ein oder mehrere Detergentien der allgemeinen Formel I.

Nach einem vierten Aspekt betrifft die vorliegende Erfindung ein Verfahren zur Bestimmung von Gesamt-Bilirubin in einer biologischen Probe nach der Diazomethode bei stark saurem pH, dadurch gekennzeichnet, dass ein oder mehrere Detergentien der allgemeinen Formel I verwendet werden.

Nach einem fünften Aspekt betrifft die vorliegende Erfindung die Verwendung eines Detergenz der allgemeinen Formel I in einem Verfahren zur Bestimmung von Gesamt-Bilirubin nach der Diazomethode bei stark saurem pH in einer biologischen Probe.

Nach einem sechsten Aspekt betrifft die vorliegende Erfindung ein Verfahren zur Bestimmung von Gesamt-Bilirubin in einer biologischen Probe nach der Diazo-Methode bei stark saurem pH nach dem Autoblanking-Verfahren, dadurch gekennzeichnet, dass ein oder mehrere Detergenzien der allgemeinen Formel I verwendet werden.

Die nachfolgenden Beispiele erläutern die Erfindung:

Beispiel 1

- Reagenslösungen:

Die Reagenslösung 1 enthält 20 g Dodecyläthyldimethylammoniumbromid und 5,195 g Sulfanilsäure aufgelöst in einem Liter 0,05 N Salzsäure. Das Reagens 2 ist eine 6,25 mmol wässrige Natriumnitritlösung. Das Reagens 1 wird mit Reagens 2 im Verhältnis 5 zu 1 gemischt. Nach 1 Minute bei RT ist das Reagensgemisch gebrauchsfertig.

- Testdurchführung auf Cobas-Bio®

Das Gebrauchsreagens wird mit Serum bzw. Bilirubinstandard bei einer determinierten Temperatur (25°C, 30°C oder 37°C), im Verhältnis 17,5:1 gemischt. Die Extinktion wird bei 550 nm genau nach 4,5 Sekunden und wieder nach 304,5 Sekunden gemessen.

- Auswertung:

- durch Vergleich der Extinktionsdifferenz mit einem Bilirubinstandard oder einem Kontrollserum:

$$\frac{(E_2P-E_1P)-RL}{(E_2S-E_1S)-RL} \times \text{Bilirubinkonzentration (S)}$$

P = Probe

S = Standard bzw. Kontrollserum.

Reagenzienleerwert (RL) wird systematisch vom Gerät gemessen und abgezogen.

- durch Faktor:

$$\text{mg/dl} = 18{,}09 \times \Delta E$$
$$\mu\text{mol/l} = 309 \times \Delta E$$

Die Abbildung 1 zeigt die Linearitätsprüfung mit einem hochkonzentrierten Bilirubinkontrollserum (42,8 mg/dl).

Beispiel 2

- Reagenslösungen

siehe Beispiel 1

- Testdurchführung: manuelles Vorgehen

In Küvetten (Schichtdicke 1 cm) werden bei Raumtemperatur die Reagenzienlösungen in der folgenden Weise pipettiert:

| | Analyse (A) | Analyseleerwert (AL) |
|---|---|---|
| - Reagenslösung 1 | - | 1,0 ml |
| - Reagensgemisch | 1,0 ml | - |
| - Probe | 0,10 ml | 0,10 ml |

Es wird gut gemischt, worauf man das Gemisch während 10 Minuten bei RT stehen lässt. In den folgenden 60 Minuten werden die Extinktion der Probe gegen Analyseleerwert bei 546 nm oder 550 nm abgelesen.

Mit frischem Reagensgemisch (1 Tag bei RT oder 5 Tage bei 4° C) kann man den Reagenzienleerwert vernachlässigen.

- Auswertung

durch Vergleich der Extinktionsdifferenz mit Bilirubinstandard oder Kontrollserum:

$$\frac{E(A)-E(AL)}{E(S)-E(SL)} \times \text{Bilirubinkonzentration (S)}$$

$$\text{durch Faktor} \quad = 12{,}4 \times \Delta E \text{ (mg/dl)}$$
$$= 212 \times \Delta E \text{ (µmol/l)}.$$

Beispiel 3

- Reagenzien

- Lyophilisiertes Diazoreagens

In eine 25 mmol wässrige Amidoschwefelsäurelösung werden der Reihe nach aufgelöst:
4 g D-Mannit
1 g Dextran 32 und
2,4 g diazotierte Sulfanilsäure, Tetrafluoboratsalz.
Die Lösung wird auf 1 Liter aufgefüllt und für die Lyophilisation in 3 ml-Portionen verteilt. Wenn die Temperatur im Lyophilisator -40° C erreicht, wird das Vakuum (0,1 mm Hg) eingestellt. Dann werden die Platten bis 10° C unter Vakuum während ca. 16 Stunden erhitzt. Schliesslich wird das Lyophilisat während 2 Stunden bei 20° C unter Vakuum getrocknet.

- Acceleratorreagens

Zu einer 0,25 M wässrigen Amidoschwefelsäurelösung werden der Reihe nach zugegeben:
75 ml Dodecyldimethylaminoxid (30% wässrige Lösung)
1 g Methylparaben und
25 g D-Mannit.
Die Lösung wird auf 1 Liter aufgefüllt.

Diese Lösung kann als flüssiges Reagens gebraucht werden oder, wie oben, nach Verteilung in 6 ml-Portionen, lyophilisiert werden.

- Gebrauchsreagenzien

Das lyophilisierte Diazoreagens wird in 3 ml entionisiertem Wasser aufgelöst. Sofern das Acceleratorreagens lyophilisiert ist, wird es in 6 ml entionisiertem Wasser aufgelöst.

- Testdurchführung auf Cobas Bio®

Die Acceleratorreagenslösung wird mit einer Serumprobe (P) oder einem Bilirubinstandard (S) bei einer determinierten Temperatur (25° C, 30° C oder 37° C) im Verhältnis 12,5:1 gemischt. Nach einer Inkubationszeit von 10 Sekunden wird die Extinktion bei 550 nm gemessen (= Analyseleerwert). Dann werden 40 Mikroliter Diazoreagenslösung in die Küvette zugegeben und die Extinktion nach 1 Sekunde und wieder nach 301 Sekunden abgelesen.

- Auswertung:

Durch Vergleich der Extinktionsdifferenz mit einem Bilirubinstandard bzw. mit einem Kontrollserum:

$$\frac{(E_2P-E_1P)-AL-RL}{(E_2S-E_1S)-SL-RL} \times \text{Bilirubinkonzentration (S)}$$

P = Probe

S = Standard

AL = Analyseleerwert, SL = Standardleerwert

RL = Reagenzienleerwert von Gerät systematisch gemessen und abgezogen.

- durch Faktor: 16,10 x ΔE für mg/dl
275 x ΔE für µmol/l

- Abbildung 2:

Kinetik der Reaktion mit einer Verdünnungsreihe von Kontrollserumgemisch (40,4 mg Bilirubin/dl).

Beispiel 4

- Reagenzien

- Lyophilisiertes Diazoreagens
- Acceleratorreagens

siehe Beispiel 3

- Gebrauchsreagenzien: siehe Beispiel 3

- Testdurchführung: manuelles Vorgehen.

In Küvetten (Schichtdicke 1 cm) werden bei Raumtemperatur die Reagenzienlösungen in der folgenden Weise pipettiert:

- Probe bzw. Standard: 0,10 ml

- Acceleratorreagenzlösung: 1,00 ml

gut mischen und Extinktion (E1) bei 546 nm oder 550 nm gegen Wasser ablesen.

- Diazoreagenslösung: 0,20 ml

gut mischen und nach 6 Minuten Extinktion (E2) gegen Wasser ablesen.

- Auswertung

- Durch Vergleich der Extinktionsdifferenz mit einem Bilirubinstandard bzw. mit einem Kontrollserum:

$$\frac{E_2P-E_1P}{E_2S-E_1S} \times \text{Bilirubinkonzentration (S)}$$

- durch Faktor: 16,3 x ΔE (mg/dl)
279 x ΔE (µmol/l)

- Abbildung 3

Richtigkeit mit Kontrollseren (Konzentration in mg/dl).

Beispiel 5

- Reagenzien

- Lyophilisiertes Diazoreagenz
- Acceleratorreagenz

siehe Beispiel 3

- Gebrauchsreagenzien

- Sofern das Acceleratorreagenz lyophilisiert ist, wird es in 6 ml entionisiertem Wasser aufgelöst.
- Das Diazoreagens wird in 18 ml Acceleratorreagenzlösung aufgelöst.

- Testdurchführung auf Cobas Bio®

Das Gebrauchsreagens wird mit Serum bzw. Bilirubinstandard bei einer determinierten Temperatur (25°C, 30°C oder 37°C), im Verhältnis 17,5:1 gemischt. Die Extinktion wird genau nach 4,5 Sekunden bei 550 nm und wieder nach 304,5 Sekunden gemessen.

- Auswertung:

- Vergleich der Extinktionsdifferenz wie in Beispiel 3
- mit Faktor = 16,80 × ΔE (mg/dl)
  = 287 × ΔE (µmol/l).

- Abbildung 4:

Kinetik der Reaktion mit der Verdünnungsreihe von einem Kontrollserumgemisch (40,4 mg/dl).

Beispiel 6

- Reagenzien siehe Beispiel 5

- Testdurchführung: (manuelles Vorgehen)

In Küvetten (Schichtdicke 1 cm) werden bei Raumtemperatur die unten angegebenen Lösungen wie folgt pipettiert:

| | Analyse (A) | Analyseleerwert (AL) |
|---|---|---|
| - Acceleratorreagenslösung | - | 1,0 ml |
| - Gebrauchsreagenslösung | 1,0 ml | - |
| - Serum bzw. Standard | 0,10 ml | 0,10 ml |

Es wird gut gemischt, worauf nach 6 Minuten die Extinktion gegen die Extinktion (AL) bei 546 nm bzw. 550 nm abgelesen wird.

Für jede Serie wird ein Reagenzienleerwert gemessen (Gebrauchsreagenzlösung).

- Auswertung

- durch Extinktionsdifferenz mnit einem Standard

$$\frac{E(A)-E(AL)-RL}{E(S)-E(SL)-RL} \times \text{Bilirubinkonzentration (S)}$$

mit Faktor: 13,20 x ΔE (mg/dl)
226 x ΔE (μmol/l).

Beispiel 7

- Lyophilisiertes Monoreagens

In eine 0,333 M wässrige Amidoschwefelsäure Lösung werden der Reihe nach zugegeben und gemischt
60 g Dodecyläthyldimethylammoniumbromid
3 g Dextran 32 und
1 g diazotierte Sulfanilsäure, Tetrafluoroboratsalz.
Die Lösung wird auf 1 Liter aufgefüllt und für die Lyophilisation in 2 ml-Portionen verteilt. Die Lyophilisation wird wie im Beispiel 3 beschrieben durchgeführt.

- Gebrauchsreagenz:

Das Lyophilisat wird in 6 ml entionisiertem Wasser aufgelöst. Dieses Reagenz ist für Analysenautomaten besonders günstig.

- Testdurchführung auf Cobas Bio®

wie im Beispiel 1 beschrieben.

- Auswertung:

- durch Vergleich mit Standard bzw. Kontrollserum siehe Beispiel 1
- Durch Faktor: 16,95 × ΔE (mg/dl)
  290 × ΔE (μmol/l).

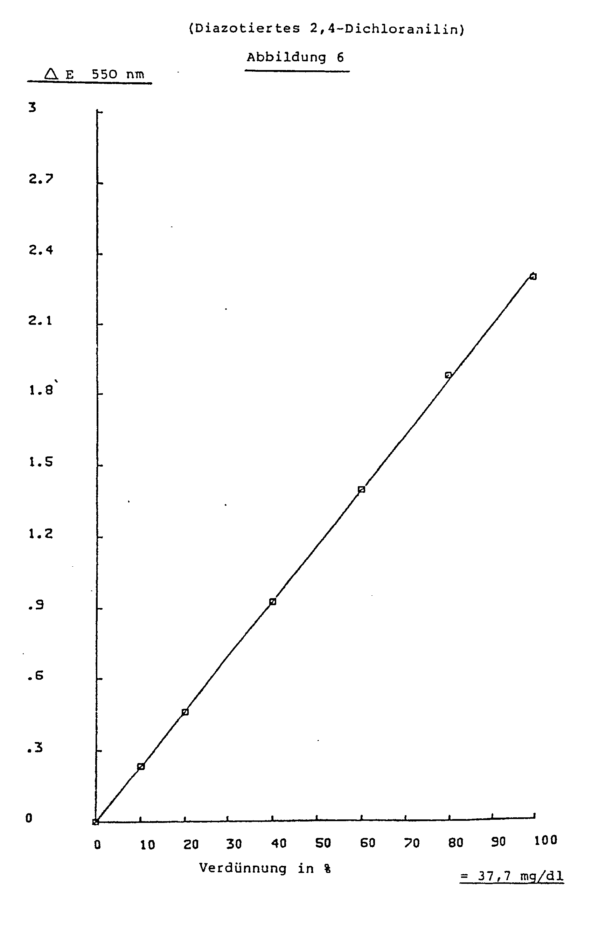

- Abbildung 5 Recovery Studie mit Kontrollserumgemischen (23-366 µmol/l).

Beispiel 8

Lyophilisiertes Monoreagens mit diazotiertem 2,4-Dichloranilin

- Lyophilisiertes Monoreagens

In eine 0,333 M wässrige Amidoschwefelsäurelösung werden der Reihe nach zugegeben und gemischt:
30 g Dodecyläthyldimethylammoniumbromid 6 g Dextran 32 und
1,4 g diazotiertes 2,4-Dichloranilin mit
Naphthalindisulfonsäure-Natriumsalz
stabilisiert.
Die Lösung wird auf 1 Liter aufgefüllt und für die Lyophilisation in 2 ml-Portionen verteilt (siehe Beispiel 3).

- Gebrauchsreagens

Das Lyophilisat wird in 6 ml entionisiertem Wasser aufgelöst.

- Testdurchführung auf Cobas-Bio®

wie in Beispiel 1 beschrieben

- Auswertung:

- durch Vergleich mit Standard bzw. Kontrollserum wie in Beispiel 1 beschrieben
- mit Faktor: 19,80 × $\Delta E$ (mg/dl)

- Abbildung 6:

Linearitätsprüfung mit einem hochkonzentrierten Bilirubinkontrollserum (37,7 mg/dl).

Beispiel 9

Lyophilisiertes Monoreagens mit diazotiertem 2,5-Dichloranilin

- Lyophilisiertes Monoreagens

In eine 0,333 M wässrige Amidoschwefelsäurelösung werden der Reihe nach zugegeben und gemischt:
30 g Dodecyläthyldimethylammoniumbromid
6 g Dextran 32 und
1,4 g diazotiertes 2,5-Dichloranilin mit
Naphthalindisulfonsäure-Natriumsalz
stabilisiert.
Die Lösung wird auf 1 Liter aufgefüllt und für die Lyophilisation in 2 ml-Portionen verteilt (siehe Beispiel 3).

- Gebrauchsreagens

Das Lysophilisat wird in 6 ml entionisiertem Wasser aufgelöst.

- Testdurchführung auf Cobas-Bio®

Das Gebrauchsreagens wird mit Serum bzw. Bilirubinstandard bei 25 °C, im Verhältnis 17,5:1 gemischt. Die Extinktion wird bei 550 nm genau nach 4,5 Sekunden und wieder nach 304,5 Sekunden gemessen.

- Auswertung

- durch Vergleich der Extinktionsdifferenz mit einem Bilirubinstandard oder einem Kontrollserum

- durch Faktor: mg/dl = 21,11 × ΔE
  µmol/l = 361 × ΔE.

Beispiel 10

Lyophilisiertes Monoreagens mit 4 Nitrobenzoldiazonium,tetrafluorboratsalz

- Lyophilisiertes Monoreagens

In eine 0,333 M wässrige Amidoschwefelsäurelösung werden der Reihe nach zugegeben und gemischt:
54 ml Dodecyldimethylamin
6 g Dextran 32
0,6 g 4 Nitrobenzoldiazonium,tetrafluorboratsalz
Die Lösung wird auf 1 Liter aufgefüllt und für die Lyophilisation in 2 ml-Portionen verteilt (siehe Beispiel 3).

- Gebrauchsreagens

Das Lyophilisat wird in 6 ml entionisiertem Wasser aufgelöst.

- Testdurchführung auf Coba-Bio®

Die Testdurchführung erfolgt wie in Beispiel 9.

- Auswertung

- durch Vergleich der Extinktionsdifferenz mit einem Bilirubinstandard oder einem Kontrollserum
- durch Faktor: mg/dl = 18,44 × ΔE
  µmol/l = 315,1 × ΔE.

## Patentansprüche

1. Wässriges Reagens zur Bestimmung von Gesamt-Bilirubin in einer biologischen Probe enthaltend einerseits
   a1) ein oder mehrere Detergenzien,
   a2) eine aromatische Aminoverbindung, die sich mit Nitrit in eine bei der Bilirubirbestimmung übliche Diazoniumverbindung überführen lässt und
   a3) eine starke Säure sowie andererseits
   b) eine wässrige Nitritlösung,
   dadurch gekennzeichnet, dass das Detergenz bzw. die Detergenzien die allgemeine Formel

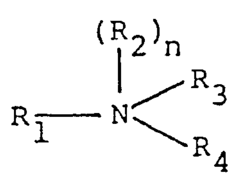

I

worin $R_1$ gerades oder verzweigtes Alkyl mit 10-16 C-Atomen bedeutet, $R_2$ Sauerstoff oder gerades oder verzweigtes Alkyl mit 1-4 C-Atomen bedeutet; $R_3$, sofern n = O ist, Wasserstoff oder gerades oder verzweigtes Alkyl mit 1-4 C-Atomen oder $-(CH_2-CH_2-O)_xH$ bedeutet, wobei x eine ganze Zahl von 1-5 ist, $R_3$, sofern n = 1 und $R_2$ Sauerstoff bedeutet, gerades oder verzweigtes Alkyl mit 1-4 C-Atomen oder $-(CH_2-CH_2-O)_xH$ bedeutet, wobei x eine ganze Zahl von 1-5 ist, $R_3$, sofern n = 1 und $R_2$ gerades oder verzweigtes Alkyl mit 1-4 C-Atomen bedeutet, gerades oder verzweigtes Alkyl mit 1-4 C-Atomen oder $-(CH_2-CH_2-O)_xH$ bedeutet, wobei x eine ganze Zahl von 1-5 ist, $R_4$, sofern n = 1 und $R_2$ Sauerstoff bedeutet, gerades oder verzweigtes Alkyl mit 1-4 C-Atomen, oder $-(CH_2-CH_2-O)_xH$ bedeutet, wobei x eine ganze Zahl von 1-5 ist, $R_4$, sofern n = 1 und $R_2$ gerades oder verzweigtes Alkyl mit 1-4 C-Atomen, gerades oder verzweigtes Alkyl mit 1-10 C-Atomen bedeutet; oder $R_2$, $R_3$ und $R_4$ zusam-

men mit dem Stickstoffatom eine Pyridiniumgruppe bilden, wobei in diesem Fall, sowie im Falle, dass n = 1 und $R_2$ gerades oder verzweigtes Alkyl mit 1-4 C-Atomen bedeutet, ein entsprechendes Anion vorhanden ist, aufweisen.

**2.** Wässriges Reagens zur Bestimmung von Gesamt-Bilirubin in einer biologischen Probe enthaltend
a) ein oder mehrere Detergenzien,
b) eine bei der Bilirubinbestimmung übliche Diazoniumverbindung und
c) eine starke Säure,
dadurch gekennzeichnet, dass das oder die Detergenzien der allgemeinen Formel I gemäss Anspruch 1 entsprechen.

**3.** Wässriges Reagens nach Anspruch 1 oder 2, dadurch gekennzeichnet, dass das Detergens der allgemeinen Formel I in einer Konzentration von 0,25 - 10% vorhanden ist.

**4.** Wässriges Reagens nach Anspruch 3, dadurch gekennzeichnet, dass das Detergens der allgemeinen Formel I in einer Konzentration von 1 - 3% vorhanden ist.

**5.** Wässriges Reagens nach Anspruch 1, dadurch gekennzeichnet, dass die aromatische Aminoverbindung in einer Konzentration von 0,5-40 mM vorhanden ist.

**6.** Wässriges Reagens nach Anspruch 5, dadurch gekennzeichnet, dass die aromatische Aminoverbindung in einer Konzentration von 1-30 mM vorhanden ist.

**7.** Wässriges Reagens nach Anspruch 1 oder 2, dadurch gekennzeichnet, dass die starke Säure Amidoschwefelsäure ist.

**8.** Wässriges Reagens nach Anspruch 7, dadurch gekennzeichnet, dass die Amidoschwefelsäure in einer Konzentration von 0\` 025-1Mol/l vorhanden ist und der pH des Reagens 1-3 ist.

**9.** Wässriges Reagens nach Anspruch 8, dadurch gekennzeichnet, dass die Amidoschwefelsäure in einer Konzentration von 0,10-0,25 Mol/l vorhanden ist und der pH des Reagens 1-3 ist.

**10.** Wässriges Reagens nach Anspruch 1 oder 2, dadurch gekennzeichnet, dass die starke Säure Salzsäure ist.

**11.** Wässriges Reagens nach Anspruch 10, dadurch gekennzeichnet, dass die Salzsäure in einer Konzentration von 0,01-0,5 Mol/l vorhanden ist und der pH des Reagens 1-3 ist.

**12.** Wässriges Reagens nach Anspruch 11, dadurch gekennzeichnet, dass die Salzsäure in einer Konzentration von 0,05-0,2 Mol/l vorhanden ist und der pH des Reagens 1-3 ist.

**13.** Wässriges Reagens nach Anspruch 1 oder 2, dadurch gekennzeichnet, dass die starke Säure Schwefelsäure ist.

**14.** Wässriges Reagens nach Anspruch 13, dadurch gekennzeichnet, dass die Schwefelsäure in einer Konzentration von 0,01-0,5 Mol/l vorhanden ist und der pH des Reagens 1-3 ist.

**15.** Wässriges Reagens nach Anspruch 14, dadurch gekennzeichnet, dass die Schwefelsäure in einer Konzentration von 0,05-0,25 Mol/l vorhanden ist und der pH des Reagens 1-3 ist.

**16.** Wässriges Reagens nach Anspruch 2, dadurch gekennzeichnet, dass die Diazoniumverbindung in einer Konzentration von 0,10-5,0 g/l vorhanden ist.

**17.** Wässriges Reagens nach Anspruch 16, dadurch gekennzeichnet, dass die Diazoniumverbindung in einer Konzentration von 0,15-3,0 g/l vorhanden ist.

**18.** Wässriges Reagens nach Anspruch 1, dadurch gekennzeichnet, dass die Nitritlösung in einer Konzentration von 4 - 100 mMol/l vorhanden ist.

19. Wässriges Reagens nach Anspruch 1 oder 2, dadurch gekennzeichnet, dass die Substituenten $R_3$ und $R_4$ in der allgemeinen Formel I die gleiche Bedeutung haben.

20. Wässriges Reagens nach Anspruch 1, dadurch gekennzeichnet, dass die aromatische Aminoverbindung 4-Aminobenzolsulfonsäure, 2,4- oder 2,5-Dichloranilin, 4-Nitroanilin, 2-Methoxy-4-nitroanilin oder 2-Methoxy-5-nitroanilin ist.

21. Wässriges Reagens nach Anspruch 2, dadurch gekennzeichnet, dass die Diazoniumverbindung diazotierte Aminobenzolsulfonsäure oder diazotiertes 2,4- oder 2,5-Dichloranilin oder diazotiertes 4-Nitroanilin oder 2-Methoxy-4-nitroanilin oder 2-Methoxy-5-nitroanilin ist.

22. Wässriges Reagens nach einem der Ansprüche 1-21, dadurch gekennzeichnet, dass das Detergens der allgemeinen Formel I N,N-Dimethyl-decylamin ist.

23. Wässriges Reagens nach einem der Ansprüche 1-21, dadurch gekennzeichnet, dass das Detergens der Formel I N,N-Dimethyl-dodecylamin ist.

24. Wässriges Reagens nach einem der Ansprüche 1-21, dadurch gekennzeichnet, dass das Detergens der Formel I N,N-Dimethyl-tetradecylamin ist.

25. Wässriges Reagens nach einem der Ansprüche 1-21, dadurch gekennzeichnet, dass das Detergens der Formel I N,N-Dimethyl-hexadecylamin ist.

26. Wässriges Reagens nach einem der Ansprüche 1-21, dadurch gekennzeichnet, dass das Detergens der Formel I Didecyldimethylammoniumchlorid ist.

27. Wässriges Reagens nach einem der Ansprüche 1-21, dadurch gekennzeichnet, dass das Detergens der Formel I Dodecyltrimethylammoniumchlorid ist.

28. Wässriges Reagens nach einem der Ansprüche 1-21, dadurch gekennzeichnet, dass das Detergens der Formel I Dodecyltrimethylammoniumbromid ist.

29. Wässriges Reagens nach einem der Ansprüche 1-21, dadurch gekennzeichnet, dass das Detergens der Formel I Dodecyläthyldimethylammoniumbromid ist.

30. Wässriges Reagens nach einem der Ansprüche 1-21, dadurch gekennzeichnet, dass das Detergens der Formel I Dodecylpyridiniumchlorid ist.

31. Wässriges Reagens nach einem der Ansprüche 1-21, dadurch gekennzeichnet, dass das Detergens der Formel I Tetradecyltrimethylammoniumbromid ist.

32. Wässriges Reagens nach einem der Ansprüche 1-21, dadurch gekennzeichnet, dass das Detergens der Formel I N,N-Dimethyldodecylaminoxid ist.

33. Stabilisiertes und lyophylisiertes Reagens zur Bestimmung von Gesamt-Bilirubin in einer biologischen Probe enthaltend diazotierte Aminobenzolsulfonsäure, diazotiertes 4-Nitroanilin, 2-Methoxy-4-nitroanilin, 2-Methoxy-5-nitroanilin, 2,4-Dichloranilin oder 2,5-Dichloranilin, sowie ein oder mehrere Detergentien der allgemeinen Formel I gemäss Anspruch 1.

34. Reagens nach Anspruch 33, dadurch gekennzeichnet, dass das Detergens der allgemeinen Formal I N,N-Dimethyldodecylamin-oxid ist.

35. Reagens nach Anspruch 33, dadurch gekennzeichnet, dass das Detergens der allgemeinen Formel I N,N-Dimethyldodecylamin ist.

36. Reagens nach Anspruch 33, dadurch gekennzeichnet, dass das Detergens der allgemeinen Formel I Dodecyläthyldimethylammoniumbromid ist.

**37.** Reagens nach Anspruch 33, dadurch gekennzeichnet, dass das Detergens der allgemeinen Formel I Dodecyltrimethylammoniumbromid ist.

**38.** Reagens nach Anspruch 33, dadurch gekennzeichnet, dass das Detergens der allgemeinen Formel I Tetradecyltrimethylammoniumbromid ist.

**39.** Reagens nach Anspruch 33, dadurch gekennzeichnet, dass das Detergens der allgemeinen Formel I Dodecylpyridiniumchlorid ist.

**40.** Verfahren zur Bestimmung von Gesamt-Bilirubin in einer biologischen Probe nach der Diazo-Methode bei stark saurem pH, dadurch gekennzeichnet, dass ein oder mehrere Detergentien gemäss allgemeiner Formel I gemäss Anspruch 1 verwendet werden.

**41.** Verfahren zur Bestimmung von Gesamt-Bilirubin in einer biologischen Probe nach der Diazo-Methode bei stark saurem pH, dadurch gekennzeichnet, dass ein oder mehrere Detergentien gemäss Ansprüchen 22-32 verwendet werden.

**42.** Verfahren nach Anspruch 40 zur Bestimmung von Gesamt-Bilirubin in einer biologischen Probe nach der Diazo-Methode bei stark saurem pH nach dem Auto-blanking-Verfahren, dadurch gekennzeichnet, dass ein oder mehrere Detergenzien der allgemeinen Formel I gemäss Anspruch 1 verwendet werden.

**43.** Verfahren nach Anspruch 41 zur Bestimmung von Gesamt-Bilirubin in einer biologischen Probe nach der Diazo-Methode bei stark saurem pH nach dem Auto-blanking-Verfahren, dadurch gekennzeichnet, dass ein oder mehrere Detergenzien gemäss Ansprüchen 22-32 verwendet werden.

**44.** Verwendung eines Detergens der allgemeinen Formel I gemäss Anspruch 1 in einem Verfahren zur Bestimmung von Gesamt-Bilirubin nach der Diazo-Methode bei stark saurem pH in einer biologischen Probe.

**45.** Verwendung von einem oder mehreren Detergenzien gemäss Ansprüchen 22-32 in einem Verfahren zur Bestimmung von Gesamt-Bilirubin nach der Diazo-Methode bei stark saurem pH in einer biologischen Probe.

## Claims

**1.** An aqueous reagent for the determination of total bilirubin in a biological sample, said reagent containing on the one hand

a1) one or more detergents,

a2) an aromatic amino compound which can be converted with nitrite into a diazonium compound which is customary in the determination of bilirubin and

a3) a strong acid, and on the other hand

b) an aqueous nitrile solution,

characterized in that the detergent or the detergents has/have the general formula

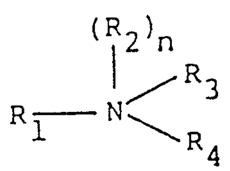

I

wherein $R_1$ signifies straight or branched alkyl with 10-16 C-atoms, $R_2$ signifies oxygen or straight or branched alkyl with 1-4 C-atoms; $R_3$, insofar as n = O, signifies hydrogen or straight or branched alkyl with 1-4 C-atoms or $-(CH_2-CH_2-O)_xH$ in which x is a whole number of 1-5, $R_3$, insofar as n = 1 and $R_2$ signifies oxygen, signifies straight or branched alkyl with 1-4 C-atoms or $-(CH_2-CH_2-O)_xH$ in which x is a whole number of 1-5, $R_3$, insofar as n = 1 and $R_2$ signifies straight or branched alkyl with 1-4 C-atoms, signifies straight or branched alkyl with 1-4 C-atoms or $-(CH_2-CH_2-O)_xH$ in which x is a whole number of 1-5, $R_4$ insofar as n = 1 and $R_2$ signifies oxygen, signifies straight or branched alkyl with 1-4 C-atoms or $-(CH_2-CH_2-O)_xH$ in which x is a whole number of 1-5, $R_4$, insofar as n = 1 and $R_2$ signifies straight or branched alkyl with 1-4 C-atoms, signifies straight or branched alkyl with 1-10 C-atoms; or $R_2$, $R_3$ and $R_4$ together with the nitrogen atom form a pyridinium group, whereby in this case as well as in the case where n = 1 and $R_2$ signifies straight or branched alkyl with 1-4 C-atoms a corresponding anion is present.

**2.** An aqueous reagent for the determination of total bilirubin in a biological sample, containing
a) one or more detergents,
b) a diazonium compound which is customary in the determination of bilirubin and
c) a strong acid,
characterized in that the detergent(s) correspond to general formula I in accordance with claim 1.

**3.** An aqueous reagent according to claim 1 or 2, characterized in that the detergent of general formula I is present in a concentration of 0. 25-10%.

**4.** An aqueous reagent according to claim 3, characterized in that the detergent of general formula I is present in a concentration of 1-3%.

**5.** An aqueous reagent according to claim 1, characterized in that the aromatic amino compound is present in a concentration of 0.5-40 mmol.

**6.** An aqueous reagent according to claim 5, characterized in that the aromatic amino compound is present in a concentration of 1-30 mmol.

**7.** An aqueous reagent according to claim 1 or 2, characterized in that the strong acid is amidosulphuric acid.

**8.** An aqueous reagent according to claim 7, characterized in that the amidosulphuric acid is present in a concentration of 0.025-1 mol/l and the pH of the reagent is 1-3.

**9.** An aqueous reagent according to claim 8, characterized in that the amidosulphuric acid is present in a concentration of 0.10-0.25 mol/l and the pH of the reagent is 1-3.

**10.** An aqueous reagent according to claim 1 or 2, characterized in that the strong acid is hydrochloric acid.

**11.** An aqueous reagent according to claim 10, characterized in that the hydrochloric acid is present in a concentration of 0.01-0.5 mol/l and the pH of the reagent is 1-3.

**12.** An aqueous reagent according to claim 11, characterized in that the hydrochloric acid is present in a concentration of 0.05-0.2 mol/l and the pH of the reagent is 1-3.

**13.** An aqueous reagent according to claim 1 or 2, characterized in that the strong acid is sulphuric acid.

**14.** An aqueous reagent according to claim 13, characterized in that the sulphuric acid is present in a concentration of 0.01-0.5 mol/l and the pH of the reagent is 1-3.

**15.** An aqueous reagent according to claim 14, characterized in that the sulphuric acid is present in a concentration of 0.05-0.25 mol/l and the pH of the reagent is 1-3.

**16.** An aqueous reagent according to claim 2, characterized in that the diazonium compound is present in a concentration of 0.10-5.0 g/l.

**17.** An aqueous reagent according to claim 16, characterized in that the diazonium compound is present in a-concentration of 0.15-3.0 g/l.

**18.** An aqueous reagent according to claim 1, characterized in that the nitrite solution is present in a concentration of 4-100 mmol/l.

19. An aqueous reagent according to claim 1 or 2, characterized in that the substituents $R_3$ and $R_4$ in general formula I have the same significance.

20. An aqueous reagent according to claim 1, characterized in that the aromatic amino compound is 4-aminobenzenesulphonic acid, 2,4- or 2,5-dichloroaniline, 4-nitroaniline, 2-methoxy-4-nitroaniline or 2-methoxy-5-nitroaniline.

21. An aqueous reagent according to claim 2, characterized in that the diazonium compound is diazotized 4-aminobenzenesulphonic acid or diazotized 2,4- or 2,5-dichloroaniline or diazotized 4-nitroaniline, 2-methoxy-4-nitroaniline or 2-methoxy-5-nitroaniline.

22. An aqueous reagent according to any one of claim 1-21, characterized in that the detergent of general formula I is N,N-dimethyl-decylamine.

23. An aqueous reagent according to any one of claim 1-21, characterized in that the detergent of formula I is N.N-dimethyl-dodecylamine.

24. An aqueous reagent according to any one of claim 1-21, characterized in that the detergent of formula I is N,N-dimethyl-tetradecylamine.

25. An aqueous reagent according to any one of claim 1-21, characterized in that the detergent of formula I is N,N-dimethyl-hexadecylamine.

26. An aqueous reagent according to any one of claim 1-21, characterized in that the detergent of formula I is didecyldimethylammonium chloride.

27. An aqueous reagent according to any one of claim 1-21, characterized in that the detergent of formula I is dodecyltrimethylammonium chloride.

28. An aqueous reagent according to any one of claim 1-21, characterized in that the detergent of formula I is dodecyltrimethylammonium bromide.

29. An aqueous reagent according to any one of claim 1-21, characterized in that the detergent of formula I is dodecylethyldimethylammonium bromide.

30. An aqueous reagent according to any one of claim 1-21, characterized in that the detergent of formula I is dodecylpyridinium chloride.

31. An aqueous reagent according to any one of claim 1-21, characterized in that the detergent of formula I is tetradecyltrimethylammonium bromide.

32. An aqueous reagent according to any one of claim 1-21, characterized in that the detergent of formula I is N,N-dimethyldodecylamine oxide.

33. A stabilized and lyophilized reagent for the determination of total bilirubin in a biological sample, containing diazotized 4-aminobenzenesulphonic acid, diazotized 4-nitroaniline, 2-methoxy-4-nitroaniline, 2-methoxy-5-nitroaniline, 2,4-dichloroaniline or 2,5-dichloroaniline, as well as one or more detergents of general formula I in accordance with claim 1.

34. A reagent according to claim 33, characterized in that the detergent of general formula I is N,N-dimethyldodecylamine oxide.

35. A reagent according to claim 33, characterized in that the detergent of general formula I is N,N-dimethyldodecylamine.

36. A reagent according to claim 33, characterized in that the detergent of general formula I is dodecylethyldimethylammonium bromide.

**37.** A reagent according to claim 33, characterized in that the detergent of general formula I is dodecyltrimethylammonium bromide.

**38.** A reagent according to claim 33, characterized in that the detergent of general formula I is tetradecyltrimethylammonium bromide.

**39.** A reagent according to claim 33, characterized in that the detergent of general formula I is dodecylpyridinium chloride.

**40.** A method for the determination of total bilirubin in a biological sample according to the diazo method at a strongly acidic pH, characterized by using one or more detergents in accordance with general formula I in accordance with claim 1.

**41.** A method for the determination of total bilirubin in a biological sample according to the diazo method at a strongly acidic pH, characterized by using one or more detergents in accordance with claims 22-32.

**42.** A method according to claim 40 for the determination of total bilirubin in a biological sample according to the diazo method at a strongly acidic pH according to the auto-blanking procedure, characterized by using one or more detergents of general formula I in accordance with claim 1.

**43.** A method according to claim 40 for the determination of total bilirubin in a biological sample according to the diazo method at a strongly acidic pH according to the auto-blanking procedure, characterized by using one or more detergents in accordance with claims 22-32.

**44.** The use of a detergent of general formula I in accordance with claim 1 in a method for the determination of total bilirubin in a biological sample according to the diazo method at a strongly acidic pH.

**45.** The use of one or more detergents in accordance with claims 22-32 in a method for the determination of total bilirubin in a biological sample according to the diazo method at a strongly acidic pH.

## Revendications

**1.** Réactif aqueux pour le dosage de la bilirubine totale dans un échantillon biologique contenant d'une part

a1) un ou plusieurs détergents,

a2) un composé aromatique aminé qui peut être converti par un nitrite en un composé de diazonium usuel pour le dosage de la bilirubine, et

a3) un acide fort, et d'autre part

b) une solution aqueuse de nitrite,

caractérisé en ce que le ou les détergents répondent à la formule générale

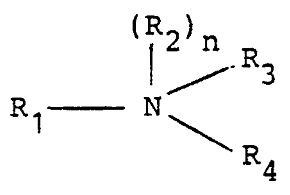

I

dans laquelle $R_1$ représente un groupe alkyle à chaîne droite ou ramifiée en C 10-C 16, $R_2$ représente l'oxygène ou un groupe alkyle à chaîne droite ou ramifiée en C 1-C 4; $R_3$, lorsque n est égal à 0, représente l'hydrogène ou un groupe alkyle à chaîne droite ou ramifiée en C 1-C 4 ou un groupe $-(CH_2-CH_2-O)_xH$ dans lequel x est un nombre entier de 1 à 5, $R_3$, lorsque n est égal à 1 et que $R_2$ représente l'oxygène, représente un groupe alkyle à chaîne droite ou ramifiée en C 1-C 4 ou un groupe $-(CH_2-CH_2-O)_xH$ dans lequel x est un nombre entier de 1 à 5, $R_3$, lorsque n est égal à 1 et que $R_2$ représente un groupe alkyle à chaîne droite ou ramifiée en C 1-C 4, représente un groupe alkyle à chaîne droite ou ramifiée en C 1-C 4 ou un groupe $-(CH_2-CH_2-O)_xH$ dans lequel x est un nombre entier

de 1 à 5, $R_4$, lorsque n est égal à 1 et que $R_2$ représente l'oxygène, représente un groupe alkyle à chaîne droite ou ramifiée en C 1-C 4 ou un groupe $-(CH_2-CH_2-O)_xH$ dans lequel x est un nombre entier de 1 à 5, $R_4$, lorsque n est égal à 1 et que $R_2$ représente un groupe alkyle à chaîne droite ou ramifiée en C 1-C 4, représente un groupe alkyle à chaîne droite ou ramifiée en C 1-C 10; ou bien $R_2$, $R_3$ et $R_4$ forment ensemble et avec l'atome d'azote un groupe pyridinium et dans ce cas, de même que dans le cas où n = 1 et $R_2$ représente un groupe alkyle à chaîne droite ou ramifiée en C 1-C 4, il y a un anion correspondant.

**2.** Réactif aqueux pour le dosage de la bilirubine totale dans un échantillon biologique contenant
a) un ou plusieurs détergents,
b) un composé de diazonium usuel pour le dosage de la bilirubine, et
c) un acide fort,
caractérisé en ce que le ou les détergents répondent à la formule générale I de la revendication 1.

**3.** Réactif aqueux selon la revendication 1 ou 2, caractérisé en ce que le détergent de formule générale I est présent à une concentration de 0,25 à 10 %.

**4.** Réactif aqueux selon la revendication 3, caractérisé en ce que le détergent de formule générale I est présent à une concentration de 1 à 3 %.

**5.** Réactif aqueux selon la revendication 1, caractérisé en ce que le composé aromatique aminé est présent à une concentration 0,5 à 40 mM.

**6.** Réactif aqueux selon la revendication 5, caractérisé en ce que le composé aromatique aminé est présent à une concentration 1 à 30 mM.

**7.** Réactif aqueux selon la revendication 1 ou 2, caractérisé en ce que l'acide fort est l'acide amidosulfurique.

**8.** Réactif aqueux selon la revendication 7, caractérisé en ce que l'acide amidosulfurique est présent à une concentration de 0,025 à 1 mole/litre et le pH du réactif est de 1 à 3.

**9.** Réactif aqueux selon la revendication 8, caractérisé en ce que l'acide amidosulfurique est présent à une concentration de 0,10 à 0,25 mole/litre et le pH du réactif est de 1 à 3.

**10.** Réactif aqueux selon la revendication 1 ou 2, caractérisé en ce que l'acide fort est l'acide chlorhydrique.

**11.** Réactif aqueux selon la revendication 10, caractérisé en ce que l'acide chlorhydrique est présent à une concentration de 0,01 à 0,5 mole/litre et le pH du réactif est de 1 à 3.

**12.** Réactif aqueux selon la revendication 11, caractérisé en ce que l'acide chlorhydrique est présent à une concentration de 0,05 à 0,2 mole/litre et le pH du réactif est de 1 à 3.

**13.** Réactif aqueux selon la revendication 1 ou 2, caractérisé en ce que l'acide fort est l'acide sulfurique.

**14.** Réactif aqueux selon la revendication 13, caractérisé en ce que l'acide sulfurique est présent à une concentration de 0,01 à 0,5 mole/litre et le pH du réactif est de 1 à 3.

**15.** Réactif aqueux selon la revendication 14, caractérisé en ce que l'acide sulfurique est présent à une concentration de 0,05 à 0,25 mole/litre et le pH du réactif est de 1 à 3.

**16.** Réactif aqueux selon la revendication 2, caractérisé en ce que le composé de diazonium est présent à une concentration de 0,10 à 5,0 g/litre.

**17.** Réactif aqueux selon la revendication 16, caractérisé en ce que le composé de diazonium est présent à une concentration de 0,15 à 3,0 g/litre.

**18.** Réactif aqueux selon la revendication 1, caractérisé en ce que la solution de nitrite est présente à une concentration de 4 à 100 millimoles/litre.

**19.** Réactif aqueux selon la revendication 1 ou 2, caractérisé en ce que les symboles $R_3$ et $R_4$ de la formule générale 1 ont la même signification.

**20.** Réactif aqueux selon la revendication 1, caractérisé en ce que le composé aromatique aminé est l'acide 4-aminobenzène-sulfonique, la 2,4- ou la 2,5-dichloraniline, la 4-nitraniline, la 2-méthoxy-4-nitrani-line ou la 2-méthoxy-5-nitraniline.

**21.** Réactif aqueux selon la revendication 2, caractérisé en ce que le composé de diazonium est le diazo de l'acide aminobenzène-sulfonique ou le diazo de la 2,4- ou de la 2,5-dichloraniline ou le diazo de la 4-nitraniline ou de la 2-méthoxy-4-nitraniline ou de la 2-méthoxy-5-nitraniline.

**22.** Réactif aqueux selon l'une des revendications 1 à 21, caractérisé en ce que le détergent de formule générale I est la N,N-diméthyl-décylamine.

**23.** Réactif aqueux selon l'une des revendications 1 à 21, caractérisé en ce que le détergent de formule I est la N,N-diméthyl-dodécylamine.

**24.** Réactif aqueux selon l'une des revendications 1 à 21, caractérisé en ce que le détergent de formule I est la N,N-diméthyl-tétradécylamine.

**25.** Réactif aqueux selon l'une des revendications 1 à 21, caractérisé en ce que le détergent de formule I est la N,N-diméthyl-hexadécylamine.

**26.** Réactif aqueux selon l'une des revendications 1 à 21, caractérisé en ce que le détergent de formule I est le chlorure de didécyldiméthylammonium.

**27.** Réactif aqueux selon l'une des revendications 1 à 21, caractérisé en ce que le détergent de formule I est le chlorure de dodécyltriméthylammonium.

**28.** Réactif aqueux selon l'une des revendications 1 à 21, caractérisé en ce que le détergent de formule I est le bromure de dodécyltriméthylammonium.

**29.** Réactif aqueux selon l'une des revendications 1 à 21, caractérisé en ce que le détergent de formule I est le bromure de dodécyléthyldiméthylammonium.

**30.** Réactif aqueux selon l'une des revendications 1 à 21, caractérisé en ce que le détergent de formule I est le chlorure de dodécylpyridinium.

**31.** Réactif aqueux selon l'une des revendications 1 à 21, caractérisé en ce que le détergent de formule I est le bromure de tétradécyltriméthylammonium.

**32.** Réactif aqueux selon l'une des revendications 1 à 21, caractérisé en ce que le détergent de formule I est l'oxyde de N,N-diméthyldodécylamine.

**33.** Réactif stabilisé et lyophilisé pour le dosage de la bilirubine totale dans un échantillon biologique contenant le diazo de l'acide aminobenzènesulfonique, le diazo de la 4-nitraniline, de la 2-méthoxy-4-nitraniline, de la 2-méthoxy-5-nitraniline, de la 2,4-dichloraniline ou de la 2,5-dichloraniline, et un ou plusieurs détergents de formule générale I de la revendication 1.

**34.** Réactif selon la revendication 33, caractérisé en ce que le détergent de formule générale I est l'oxyde de N,N-diméthyldodécylamine.

**35.** Réactif selon la revendication 33, caractérisé en ce que le détergent de formule générale I est la N,N-diméthyldodécylamine.

**36.** Réactif selon la revendication 33, caractérisé en ce que le détergent de formule générale I est le bromure de dodécyléthyldiméthylammonium.

**37.** Réactif selon la revendication 33, caractérisé en ce que le détergent de formule générale I est le bromure de dodécyltriméthylammonium.

**38.** Réactif selon la revendication 33, caractérisé en ce que le détergent de formule générale I est le bromure de tétradécyltriméthylammonium.

**39.** Réactif selon la revendication 33, caractérisé en ce que le détergent de formule générale I est le chlorure de dodécylpyridinium.

**40.** Procédé pour doser la bilirubine totale dans un échantillon biologique par le mode opératoire au diazo à pH fortement acide, caractérisé en ce que l'on utilise un ou plusieurs détergents de formule générale I de la revendication 1.

**41.** Procédé pour doser la bilirubine totale dans un échantillon biologique par le mode opératoire au diazo à pH fortement acide, caractérisé en ce que l'on utilise un ou plusieurs détergents selon les revendications 22 à 32.

**42.** Procédé selon la revendication 40, pour doser la bilirubine totale dans un échantillon biologique par le mode opératoire au diazo à pH fortement acide selon la technique d'auto-étalonnage, caractérisé en ce que l'on utilise un ou plusieurs détergents de formule générale I de la revendication 1.

**43.** Procédé selon la revendication 41, pour doser la bilirubine totale dans un échantillon biologique par le mode opératoire au diazo à pH fortement acide selon la technique d'auto-étalonnage, caractérisé en ce que l'on utilise un ou plusieurs détergents selon les revendications 22 à 32.

**44.** Utilisation d'un détergent de formule générale I de la revendication 1 dans un procédé pour le dosage de la bilirubine totale par le mode opératoire au diazo à pH fortement acide dans un échantillon biologique.

**45.** Utilisation d'un ou plusieurs détergents selon revendications 22 à 32, dans un procédé pour le dosage de la bilirubine totale par le mode opératoire au diazo à pH fortement acide dans un échantillon biologique.

Abbildung 1

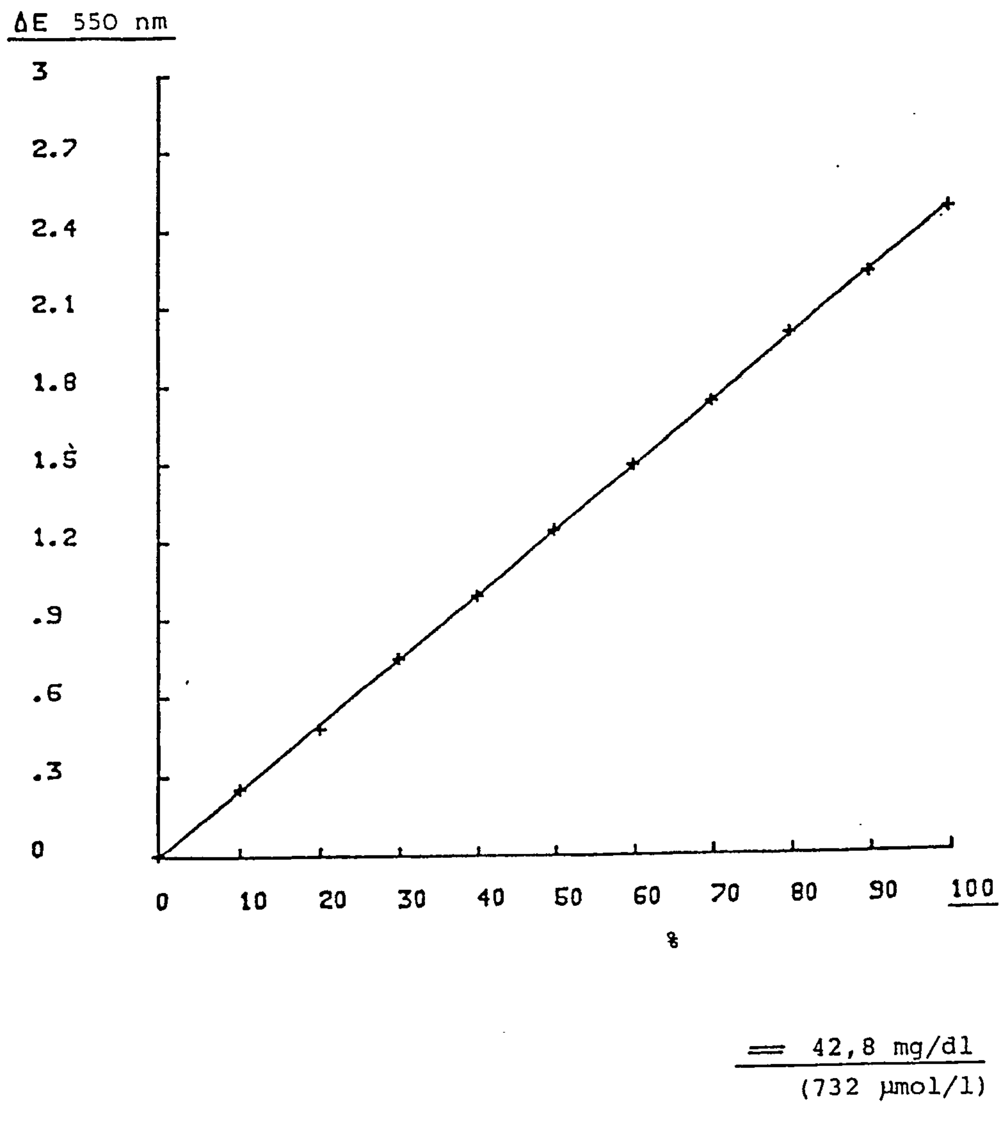
ΔE 550 nm
3
2.7
2.4
2.1
1.8
1.5
1.2
.9
.6
.3
0
0
10
20
30
40
50
60
70
80
90
100
%
≙ 42,8 mg/dl
(732 µmol/l)

Kinetik bei Analysentyp 6

Abbildung 2

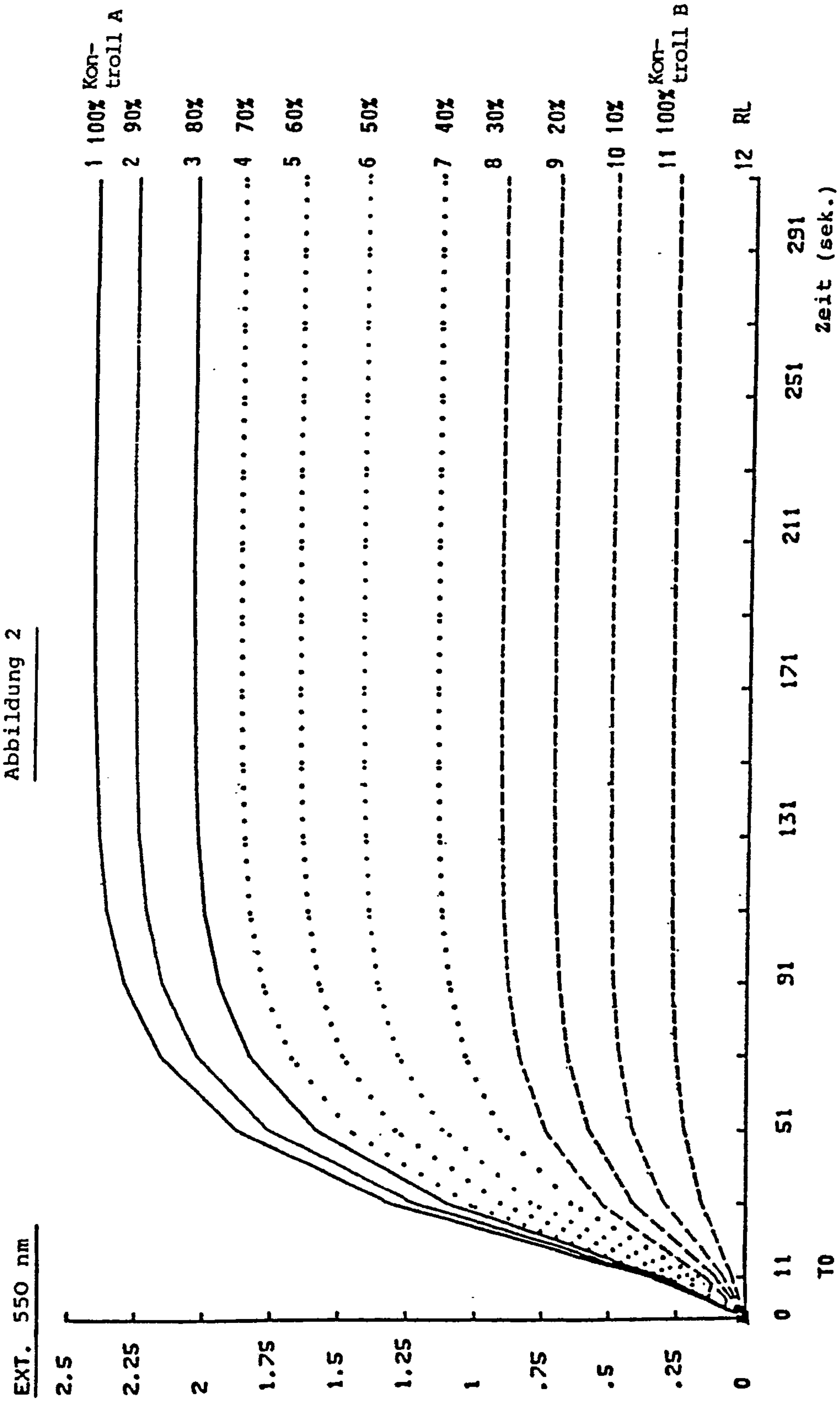

## Abbildung 3

Manuelle Methode: Uebereinstimmung mit Kontrollseren

| Kontrollseren | Erklärter Wert | Ergebnis |
|---|---|---|
| N <Roche> P 1833 | 1.2 - 1.4 - 1.6 | 1.50 |
| P <Roche> E 0641 | 3.0 - 3.2 - 3.4 | 3.20 |
| Moni-Trol I-E LTD 205 | 1.35- 1.46- 1.57 | 1.46 |
| Moni-Trol II-E PTD 103 | 3.6 - 3.94- 4.3 | 4.10 |
| Validate N 4993073 | 0.96- 1.20- 1.44 | 1.16 |
| Validate A 4662033 | 3.40- 4.20- 5.00 | 4.54 |
| BIC 969 R | 18.6 -20.4 -22.2 | 20.20 |
| Kontrollogen 613412 | 17.2 -20.2 -23.2 | 19.40 |

Abbildung 4

Kinetik bei Analysentyp 5

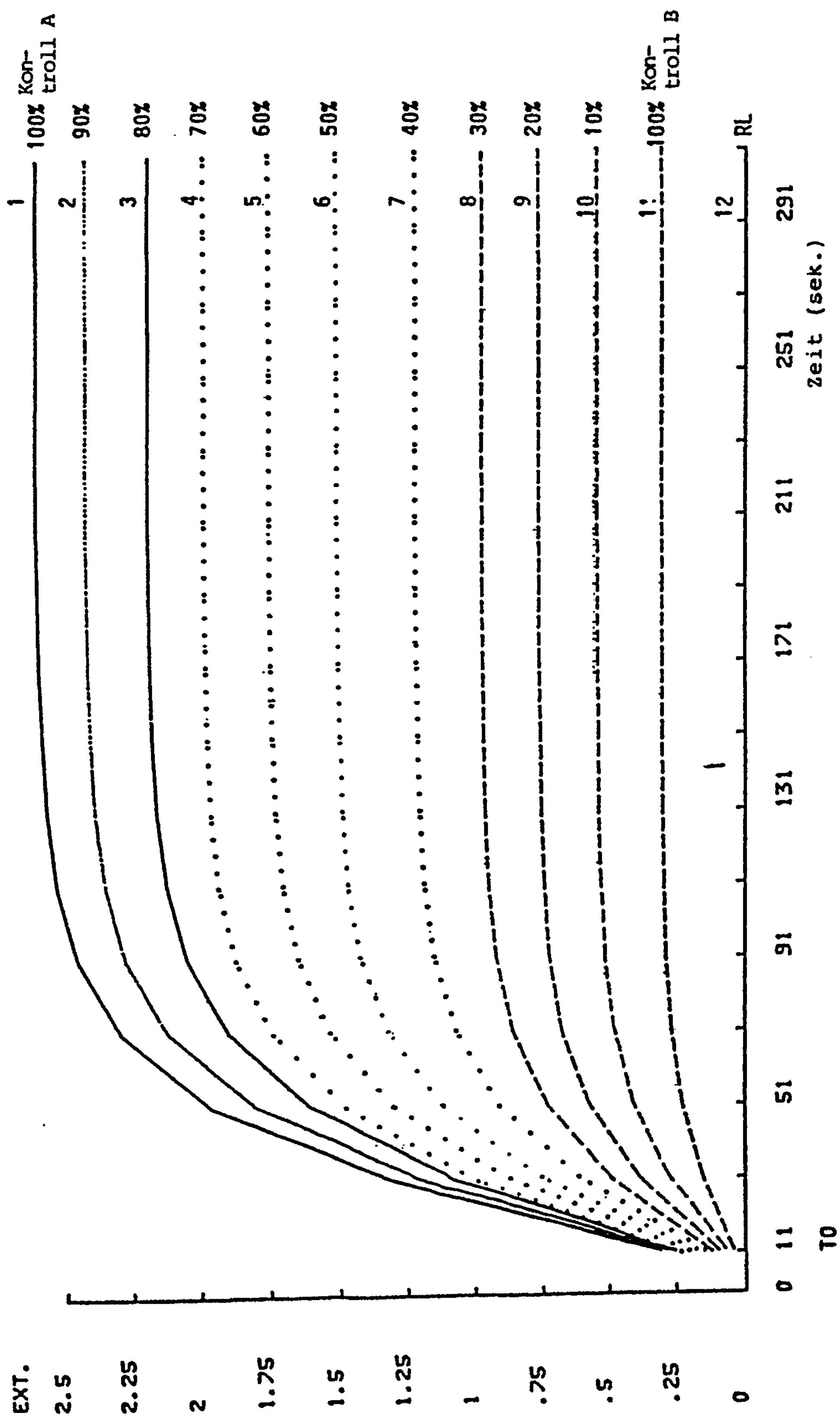

Abbildung 5

Recovery-Studie

| | | erklärter Wert µmol/l | gemessener Wert µmol/l | Recovery % |
|---|---|---|---|---|
| 1. Kontrollogen Bilirubin Lot A613413A | + Sigma Standardset 34F6094 | | | |
| 100 % | 0 % | 365.9 | 362.9 | 99.2 |
| 80 % | 20 % | 326.9 | 323.5 | 99.0 |
| 60 % | 40 % | 287.9 | 286.6 | 99.5 |
| 40 % | 60 % | 248.9 | 248.3 | 99.8 |
| 20 % | 80 % | 209.9 | 211.1 | 100.5 |
| 0 % | 100 % | 171.0 | 176.0 | 102.9 |
| | | | | Mittel: 100.1% |
| 2. 〈Roche〉 N, Lot P1039 | + Sigma Standardset 34F6094 | | | |
| 0 % | 100 % | 171.0 | 174.4 | 101.9 |
| 20 % | 80 % | 141.9 | 144.0 | 101.4 |
| 40 % | 60 % | 111.8 | 112.8 | 100.9 |
| 60 % | 40 % | 82.3 | 82.6 | 100.3 |
| 80 % | 20 % | 52.7 | 53.0 | 100.5 |
| 100 % | 0 % | 23.1 | 23.9 | 101.7 |
| | | | | Mittel: 101.1% |

(Diazotiertes 2,4-Dichloranilin)

Abbildung 6